Europäisches Patentamt

European Patent Office

Office européen des brevets

(19)

(11) Numéro de publication: **0 275 222**
**A2**

# (12) DEMANDE DE BREVET EUROPEEN

(21) Numéro de dépôt: **88400037.3**

(22) Date de dépôt: **08.01.88**

(51) Int. Cl.⁴: **A 61 K 31/19**
**A 61 K 31/215**

(30) Priorité: **15.01.87 FR 8700371**

(43) Date de publication de la demande:
**20.07.88 Bulletin 88/29**

(84) Etats contractants désignés:
**BE CH DE ES FR GR IT LI LU**

(71) Demandeur: **Froment, Suzanne**
**47 bis, rue Monte-Carlo**
**F-13000 Marseille (FR)**

**Veyron, Hélène**
**2, place Foch**
**F-13004 Marseille (FR)**

**Giustiniani, Virginie**
**9E, rue de Provence**
**F-13004 Marseille (FR)**

(72) Inventeur: **Froment, Suzanne**
**47 bis, rue Monte-Carlo**
**F-13000 Marseille (FR)**

**Veyron, Hélène**
**2, place Foch**
**F-13004 Marseille (FR)**

**Giustiniani, Virginie**
**9E, rue de Provence**
**F-13004 Marseille (FR)**

(74) Mandataire: **Clisci, Serge**
**S.A. FEDIT-LORIOT 38 avenue Hoche**
**F-75008 Paris (FR)**

(54) Utilisation de l'acide glycyrrhétinique en tant que moyen cicatrisant.

(57) La présente invention a trait à une nouvelle utilisation en thérapeutique par voie locale, en tant que moyen cicatrisant, d'une substance appartenant à l'ensemble comprenant l'acide glycyrrhétinique, notamment l'acide 18β - glycyrrhétinique, et ses dérivés, notamment ses sels non-toxiques et ses esters non-toxiques.

EP 0 275 222 A2

Bundesdruckerei Berlin

**Description**

## UTILISATION DE L'ACIDE GLYCYRRHETINIQUE EN TANT QUE MOYEN CICATRISANT

La présente invention a trait à une nouvelle utilisation en tant que moyen cicatrisant, d'une substance déjà connue en thérapeutique en tant que produit analgésique et antiinflammatoire, à savoir l'acide glycyrrhétinique.

On sait notamment des brevets français N° 1306 M et N° 3810 M et du certificat d'addition N° 40 CAM, que l'acide glycyrrhétinique a déjà été préconisé en tant que principe actif analgésique et antiinflammatoire. On sait en particulier qu'il intervient en tant que moyen analgésique et antiinflammatoire dans une formulation de pâte gingivale commercialisée sous le nom de marque d'ARTHRODONT (voir à cet effet. le Dictionnaire Vidal 61ème édition, page 107, OVP, Paris, 1985).

On vient de trouver de façon surprenante que (i) l'acide glycyrrhétinique, et (ii) ses dérivés (notamment ses sels et esters) présentent par voie locale des propriétés cicatrisantes particulièrement intéressantes.

Suivant l'invention on préconise donc une nouvelle utilisation d'une substance glycyrrhétinique choisie parmi l'ensemble comprenant l'acide glycyrrhétinique, ses sels non toxiques et ses esters non toxiques, pour l'obtention d'un médicament cicatrisant destiné à une utilisation thérapeutique locale vis-à-vis des plaies.

L'acide glycyrrhétinique peut être indifféremment l'isomère 18β ou l'isomère 18α ; par commodité on préfère néanmoins utiliser plutôt l'acide β - glycyrrhétinique.

Parmi les esters qui conviennent suivant l'invention on peut notamment citer les glycyrrhétinates d'alkyle en $C_1$-$C_4$, de phényle et de benzyle.

Parmi les sels non-toxiques qui conviennent suivant l'invention on peut mentionner en particulier les sels métalliques, notamment les sels de sodium et de potassium, et les sels d'addition avec des bases organiques.

Le meilleur mode de mise en oeuvre de l'invention consiste à utiliser l'acide glycyrrhétinique pur et en particulier l'acide 18β - glycyrrhétinique.

De façon pratique, pour la nouvelle utilisation locale suivant l'invention, on préconise une composition thérapeutique qui renferme une substance cicatrisante choisie parmi l'acide glycyrrhétinique (de préférence l'acide 18β - glycyrrhétinique), ses sels non-toxiques et ses esters non-toxiques, en association avec un excipient approprié physiologiquement acceptable.

Bien entendu dans une telle composition, le principe actif cicatrisant intervient à une teneur pharmaceutiquement efficace pour la cicatrisation des plaies.

Selon un mode particulier de mise en oeuvre on recommande une composition thérapeutique qui renferme l'acide glycyrrhétinique, un agent antiinfectieux tel que le formaldéhyde et un excipient approprié pour application locale, notamment sous forme de crème, pâte, pommade, onguent, lotion, etc...

D'une manière générale les compositions thérapeutiques suivant l'invention renfermeront de 0,5 à 5 % en poids d'acide glycyrrhétinique ou d'un de ses sels ou esters non-toxiques par rapport au poids total desdites compositions. Si le formaldéhyde est incorporé dans ces compositions, il le sera à une teneur de 0,05 à 0,5 % en poids par rapport au poids total desdites compositions.

De façon avantageuse, lesdites compositions thérapeutiques contiendront 1g d'acide glycyrrhétinique, et le cas échéant 0,1g de formaldéhyde, pour 100g desdites compositions.

D'autres avantages et caractéristiques de l'invention seront mieux compris à la lecture, qui va suivre, de résultats d'essais pharmacologiques et cliniques entrepris avec l'acide glycyrrhétinique en tant que moyen cicatrisant, l'ensemble de ces éléments n'étant pas limitatif mais donné à titre d'illustration.

## A. ESSAIS PHARMACOLOGIQUES

Les propriétés cicatrisantes de l'acide glycyrrhétinique ont été étudiées chez le rat mâle adulte pesant de 180 à 200g. Après avoir rasé le dos le long de la colonne vertébrale et provoqué une plaie standardisée de 1 cm$^2$ par brûlure au moyen d'un poinçon approprié porté à 180° C, des lots de 10 rats reçoivent une application locale quotidienne sur ladite blessure d'un excipient (lot témoin) ou d'une composition (lot traité) comprenant ledit excipient (99 parties en poids) et l'acide 18β - glycyrrhétinique pur (1 partie en poids), pendant 2 semaines.

On constate que le lot traité cicatrise plus rapidement que le lot témoin et que l'acide glycyrrhétinique est bien toléré par l'animal.

## B. ESSAIS CLINIQUES

On prélève des greffons pour réaliser deux autogreffes gingivales successives. On obtient deux plaies expérimentales identiques au niveau palatin droit et gauche, chez la même patiente à environ un mois d'intervalle.

L'une des deux plaies permet d'apprécier l'activité de l'onguent gingival qui renferme l'acide 18β -glycyrrhétinique par comparaison avec l'autre plaie traitée par le même onguent ne renfermant pas ledit acide (placebo)

### 1) - Choix des patients

On a fait appel à des patientes volontaires agées de 38 à 47 ans ne présentant aucune maladie intercurrente succeptible d'influencer favorablement ou négativement la cicatrisation, ayant des conditions anatomiques identiques des deux côtés du palais et s'engageant à n'accepter que l'onguent gingival sus-visé avec ou sans acide 18β - glycyrrhétinique et, le cas échéant, un analgésique destiné à diminuer la douleur à compter du premier jour de l'essai.

2) - Chronologie de l'étude

Une plaque palatine de protection (résine autopolymérisante) est réalisée avant la première intervention, chez les patientes prises comme témoins on prélève au bistouri à l'instant J = O, un greffon ayant approximativement les dimensions suivantes : longueur : 20 mm, largeur : 8 mm, épaisseur : 1,5 mm, sur un côté du palais (droit ou gauche, indifféremment), au niveau de la muqueuse palatine, et plus précisément au voisinage de la première molaire supérieure, à environ 2 mm du collet.

Ce prélèvement est effectué en vue de la réalisation d'une autogreffe du côté opposé du palais.

Après le prélèvement du greffon, une hémostase est pratiquée par compression au moyen d'une gaze, et la plaque palatine est mise en place avec l'onguent, au niveau de la plaie.

Il s'agit de l'onguent placebo qui est constitué par l'excipient de la formulation de traitement donnée ci-après. Ensuite, la patiente n'est autorisée à absorber que des comprimés analgésiques. Elles est priée de renouveler l'onguent sous la plaque palatine dans les trois heures, le jour de l'intervention, puis dès le lendemain jusqu'au jour J + 8 trois fois par jour avec, le cas échéant, un comprimé analgésique en cas de douleur.

A l'instant J + 8, on apprécie l'état de cicatrisation de la plaie palatine par étude cytologique et mesure de la sérosité.

On note également que le nombre de comprimés analgésiques (notamment de 4-hydroxyacétanilide, de galfénine, etc...) pris par le patient, l'aspect clinique de la plaie et les éventuelles réactions du patient.

La sérosité au niveau de la plaie palatine est quantifiée selon la méthode décrite par FOUREL et al., Journal de Parodontologie 4, 391-398, (1985).

Après séchage à l'aide de compresses de gaze, la plaie est aséchée par un jet d'air agissant pendant 10 secondes à une distance standardisée (la patiente ayant la bouche au maximum d'ouverture, l'embout de la seringue à air étant placé au niveau de l'embrasure vestibulaire située entre la 2ème prémolaire et la 1ère molaire mandibulaires du même côté que la plaie). Une bande (en anglais : "strip") de papier buvard("Perio-paper"Har-601) est aussitôt placée sur la plaie où il tient par adhésion pendant 30 secondes ; son degré d'humidité est aussitôt évalué à l'aide d'un appareil Periotron (modèle 600).

L'étude cytologique a été réalisée comme suit. Un filtre Millipore®(type HA - 0,45 µm) est placé à cheval sur la plaie et la muqueuse palatine environnante. Après décollement, il est fixé au "Cytospray" et numéroté. Les filtres sont colorés au laboratoire selon la méthode de LAINSON et MACKENZIE (Journal of Periodontology 47 : 469-476 (1976)] .

L'interprétation des échantillons est faite en considérant les éléments suivants :
- cellules basophiles et éosinophiles
- cellules acidophiles
- cellules pycnotiques
- cellules kératinisées
- flore

COTE EXPERIMENTAL

Un mois environ après la première, une seconde autogreffe est pratiquée chez le même patient. Le prélèvement du greffon palatin est fait du côté opposé au au précédent prélèvement.

Le protocole expérimental est exactement le même que du côté témoin, sauf que l'onguent utilisé répond à la formulation suivante :
- acide 18β - glycyrrhétinique...1 g
- soluté officinal de formaldéhyde...0,10 g
- rongalite...0,05 g
- excipient q.s.p. 100 g

L'excipient utilisé comprend en association pour réaliser un onguent de l'alginate de sodium, de la gomarine, du silicate de sodium, du texapon, de la glycérine, du carbonate de calcium, de l'eugénol, du menthol, de l'essence de menthe et de l'eau bidistillée.

Bien entendu, toutes les plaies sont revues au moins un mois après les interventions.

3)- Résultats

L'étude a porté sur huit plaies expérimentales soit huit autogreffes gingivales, qui ont été suivies chez quatre patientes.

a. Apprécitation clinique

Les patientes ont pris moins de comprimés analgésiques quand elles utilisaient l'onguent renfermant l'acide glycyrrhétinique au lieu du placebo (15 comprimés au total pour les quatre patientes lors de la prise du placebo et 8 comprimés au total pour les patientes lors de la prise de l'onguent gingival de traitement).

On constate qu'à l'instant J + 8 l'aspect des plaies a été jugé satisfaisant, du côté de l'onguent gingival de traitement et un peu moins satisfaisant du côté placebo. A l'instant J + 30, les huit plaies ont cicatrisé sans aucune complication.

Lors de contrôles effectués ultérieurement, il a été constaté que la muqueuse palatine avait retrouvé son intégrité normale.

b. Quantification de la sérosité

Chez les quatre patientes, la sérosité a toujours été moins abondante à J + 8, du côté traité par l'onguent gingival renfermant l'acide 18β - glycyrrhétinique que du côté recevant le placebo. Les résultats qui figurent dans le tableau I ci-après, ont trait au degré d'humidité de la bande de papier mesuré au Périotron® 600.

TABLEAU I

|  | Placebo | acide 18β-glycyrrhétinique |
|---|---|---|
| Patient 1 | 82 | 66 |
| Patient 2 | 67 | 56 |
| Patient 3 | 87 | 74 |
| Patient 4 | 69 | 53 |

c. Etude cytologique à J + 8

On constate que du côté placebo, il existe une zone inflammatoire et infectée alors que du côté de l'onguent gingival renfermant l'acide 18β - glycyrrhétinique, il n'y a ni inflammation ni infection, à l'instant J +8.

d. Conclusions

On constate que l'acide 18β - glycyrrhétinique est parfaitement toléré dans le protocole expérimental sus-visé, qu'il manifeste son action analgésique dès les 24 premières heures de la cicatrisation, qu'il favorise de façon bénéfique ladite cicatrisation en asséchant les plaies eu égard de la diminution de la sérosité à J + 8. Son action positive sur la cicatrisation est confirmée par l'étude cytologique à J + 8.

**Revendications**

1. Nouvelle utilisation d'une substance glycyrrhétinique, caractérisée en ce que l'on utilise une substance glycyrrhétinique choisie parmi l'ensemble comprenant l'acide glycyrrhétinique, ses sels non-toxiques et ses esters non-toxiques, pour l'obtention d'un médicament cicatrisant destiné à une utilisation thérapeutique locale vis-à-vis des plaies.

2. Utilisation suivant la revendication 1, caractérisée en ce que la substance glycyrrhétinique est l'acide 18β - glycyrrhétinique.

3. Utilisation suivant la revendication 1, caractérisée en ce que la substance glycyrrhétinique est l'acide 18α - glycyrrhétinique.

4. Utilisation suivant la revendication 1, caractérisée en ce que la substance glycyrrhétinique est choisie parmi les glycyrrhétinates d'alkyle en $C_1$ -$C_4$, de phényle et de benzyle.

5. Utilisation suivant la revendication 1, caractérisée en ce que la substance glycyrrhétinique est choisie parmi les sels de sodium et de potassium de de l'acide glycyrrhétinique.

6. Composition thérapeutique cicatrisante, caractérisée en ce qu'elle renferme, en association avec un excipient approprié à l'usage local et physiologiquement acceptable, une substance cicatrisante, choisie parmi l'acide glycyrrhétinique, de préférence l'acide 18β -glycyrrhétinique , ses sels non-toxiques et ses esters non-toxiques.

7. Composition suivant la revendication 1, caractérisée en ce qu'elle renferme 0,5 à 5 % en poids d'acide glycyrrhétinique ou d'un de ses sels ou esters non-toxiques par rapport au poids total de ladite composition.

8. Composition suivant la revendication 6, caractérisée en ce que la substance glycyrrhétinique est associée à du formaldéhyde.

4

9. Composition suivant la revendication 8, caractérisée en ce qu'elle renferme pour 100 g, 1 gramme d'acide 18β - glycyrrhétinique et 0,1 g de formaldéhyde.